# EUROPÄISCHE PATENTANMELDUNG

(11) **EP 2 808 059 A1**
(43) Veröffentlichungstag der Anmeldung: **03.12.2014**
(21) Anmeldenummer: 13169770.8
(22) Anmeldetag: 29.05.2013
(51) Int. Cl.: A61Q 19/10, A61K 8/92, A61K 8/96, A61K 8/02, A61K 8/19

(54) **2- Komponenten Badezusatz mit Matrix-abhängiger zeitverzögerter Farbstoff-Freisetzung**

(71) Anmelder: Merz Pharma GmbH & Co. KGaA, 60318 Frankfurt am Main (DE)
(72) Erfinder: Hönkemeyer, Nadine, 60486 Frankfurt/Main (DE); Becker, Bärbel, 65719 Hofheim (DE); Berlekamp, Dr., Uwe, 48432 Rheine (DE)
(74) Vertreter: Müller, Claudia

(57) **Zusammenfassung**

Die vorliegende Erfindung betrifft einen 2- Komponenten- Kombinations-Badezusatz auf Basis eines wasserlöslichen Trägers mit zeitverzögerter Freisetzung der vorhandenen Wirkstoffe, insbesondere der Farbstoffe. Dies erfolgt durch die Kombination zweier Formkörperkomponenten 1, 2 mit unterschiedlich gestalteten Matrices: die erste Matrix umfasst kristallline lose Formkörper 1 wie Pulver, Granulat, die zweite Matrix umfasst feste, insbesondere gepresste Formkörper 2 wie Tabletten. Beide Formkörperkompo-nenten umfassen voneinander verschiedene Farbstoffe. Aufgrund der physikalischen Beschaffenheit der Matrices werden bei Anwendung des Kombinations-Badezusatzes zunächst die Partikel der ersten Formkörperkomponente, vor allem Träger und darin vorhandene Farbstoffe herausgelöst. Anschließend werden zeitversetzt die Partikel, insbesondere Träger, Farbstoffe der Formkörperkomponente 2 herausgelöst. Diese Zeitverzögerung erfolgt überaschenderweise ohne die Anwesenheit von bisher erforderlichen, eine Trägerphase umhüllenden Retardierungsmitteln wie Schellack oder Wachse.

## Beschreibung

Die vorliegende Erfindung betrifft einen 2- Komponenten-Kombinations-Badezusatz auf Basis eines wasserlöslichen Trägers mit zeitverzögerter Freisetzung der vorhandenen Wirkstoffe, insbesondere der Farbstoffe. Dies erfolgt durch die Kombination zweier unterschiedlich gestalteter Trägermatrices: die erste Matrix besteht aus kristallinen, insbesondere grob-kristallinen losen, schüttbaren Formkörpern aus wasserlöslichen Trägerpartikeln einer Korngröße von ca. 1 - 4 mm, die zweite Matrix besteht aus gepress-ten Formkörpern aus insbesondere feinkörnigem Trägermaterial z. B. mit einer Korn-größe von 0,2 - ca. 1 mm auf Salzbasis. Die erste Matrix umfasst einen oder mehrere Farbstoffe der Wirkstoffgruppe 1, und weiterhin ggf. Zusatzstoffe, ausgewählt aus Reinigungsstoffen, (Emulgatortensiden), Zusatzwirkstoffen wie Lipiden, galenischen Zusatzstoffen oder Mischungen hiervon. Die zweite Matrix umfasst eine Wirkstoffgruppe 2 aus Lipid(en), insbesondere flüssigen(m) Öl(en), Farbstoffen, weiterhin ggf. Zusatzstoffe, ausgewählt aus Zusatzwirkstoffen, galenischen Zusatzstoffen oder Mischungen hiervon. Die Trägerpartikel der ersten und zweiten Matrix können gleich oder verschie-den sein. Aufgrund der physikalischen Beschaffenheit der Matrices werden bei An-wendung zunächst die Partikel der ersten Matrix, also Träger und darin vorhandene Farbstoffe, ggf. Zusatzstoffe der Wirkstoffgruppe 1 herausgelöst. Aufgrund der sub-stantiellen Beschaffenheit der beiden Matrices, insbesondere auch durch die Anwe-senheit eines oder mehrerer, auf den Partikeln aufgetragener, flüssiger Öle in der Matrix (Struktur) der zweiten Komponente erfolgt ggf. auch in Abhängigkeit der gewählten Korngröße und/oder des Verpressungsgrades ein zeitversetztes Herauslösen der dorti-gen Farbstoffe der Wirkstoffgruppe 2. Dadurch ist es möglich, die mittels der Matrix 1 hergestellte Färbung der Badeflotte nach einem bestimmten Zeitpunkt zu wechseln. Zusätzlich kann die mittels Wirkstoffgruppe 1 behandelte und vor allem gereinigte Haut noch während des Badevorgangs auch mit den flüssigen Ölen (Pflegestoffen) versorgt werden, um so einen Ausgleich bzgl. des Lipid- und Feuchtigkeitsgehalts der Haut zu erzielen. Die Zeitverzögerung erfolgt überraschenderweise ohne die Anwesenheit von bisher erforderlichen, eine Partikelphase umhüllenden Retardierungsmitteln wie Schellack oder Wachse. Dadurch kann auf derartige Zusätze verzichtet werden. Durch Wahl geeigneter unterschiedlicher Farb- und/ oder ggf. Wirkstoffe wie ätherische Öle (Aromen) kann ein zeitlicher Farb- ggf. auch ein Aromawechsel erzielt werden, so dass für den Anwender die jeweilige Badephase wie Reinigung bzw. Pflege visuell und ggf. auch geruchlich (aroma-spezifisch) erkennbar wird.

### Stand der Technik

Badezusätze sind in unterschiedlichsten Ausführungsformen seit langem bekannt. Sie können als flüssige Zusätze, z. B. in Form von schäumenden Emulsionen oder Ölen, oder als feste Zusätze, z. B. als salzhaltige Feststoffe, sowohl kosmetisch als auch therapeutisch eingesetzt werden. Durch Wahl geeigneter Zusatzstoffe können sowohl physiologische Effekte wie Beruhigung, Hautpflege, Hauttherapie (z.B. dermatische Störungen), Entspannung, Belebung, Entzündungshemmung u.a. als auch visuelle Effekte durch entsprechende Farbgebung erreicht werden. Feste Badezusätze sind insbesondere im Hinblick auf Transportaspekte besonders günstig. Dabei handelt es sich überwiegend um Zusätze auf Basis von Salz wie Meersalz und Steinsalz.

So beschreibt die EP 0930 064 B1 Badezusätze in Form von Pulvern oder Granulaten, welche einen Mineralsalzgehalt, sowie weiterhin höhere Menge von bis zu 50 Gew.% insbesondere feste Lipidkomponenten wie hydrierte Öle oder Glyceride, und Tenside sowie vesikelbildende Komponenten aufweisen. Damit können liposomale Strukturen trotz des Vorhandenseins von Tensiden und großer Mengen Elektrolyten (Salze) entstehen. Darüber hinaus können vor allem Carbonatsalze mit einem zusätzlichen belebenden Sprudeleffekt vorhanden sein.

Die WO 2011/006616 betrifft u.a. feste Badezusätze, welche ein Prämix-Konzentrat aus Lipiden, Tensiden, liposomenbildenden Phospholipiden und Proteinen aufweisen. Letztere zwei Komponenten bilden bei Kontakt mit einem Überschuss an Wasser unmittelbar hautaktive Proteoliposomen (Proteosomen) mit katalytischer Aktivität für einen verbesserten Wirkstofftransport in die Haut.

Die WO 2010/022873 A2 beschreibt Badeperlen aus 80 bis 97 Gew.% eines festen Trägers wie Kollagenhydrolysat, Gelatine oder Gelatinehydrolysat, Cellulose, Kohlenhydrate, Stärke oder Harnstoff mit bis zu 10% Lipiden, und bis zu 11 Gew.% Tensiden sowie liposomenbildenden Substanzen. Hierbei sind die Träger nicht salzartiger Natur, sondern kolloidal und weisen daher auch gute Adsorptionseigenschaften auf. Darüber hinaus werden liposomale Strukturen zur Wirkverbesserung eingesetzt.

Eine andere Wirkung als die physiologische ist in der DE 10 2009 031 273 A1 beschrieben: hierin werden feste Badezusätze auf Salzbasis offenbart, welche insbesondere akustische Effekte auslösen. Die beschriebenen Badezusätze in fester Form weisen einen Gehalt an Zucker wie Glucose und/oder Saccharose, Lactose, sowie Farbstoffe, Pflegestoffe, Parfüm- und/oder Aromastoffe, und ad 100 Gew.% Salz auf. Dabei soll der akustische Effekt durch den Zucker erreicht werden, der knisternd gleichzeitig mit den übrigen Substanzen in Lösung geht. Ein solcher Badezusatz eignet sich daher insbesondere zur Anwendung bei Kindern.

Die DE 000001061965 B betrifft feste Sprudelbadezusätze, die statt einer grob kristallinen, pulverförmigen oder gepressten scheibenförmigen Ausgestaltung kegelförmig, zylinderartig oder ähnlich ausgebildet sind. Durch diese Formkörper verläuft ein Kanal, durch den Wirkstoffe langsam austreten. Weiterhin ist dieser Formkörper von außen mit einer Schicht versehen, die bewirkt, dass die im Inneren befindlichen Wirkstoffe einschließlich Kohlendioxid nur aus dem Lösungskanal austreten.

DE 102011009633 A1 beschreibt einen festen körnigen Badezusatz auf Salzbasis, bestehend aus unterschiedlich beladenen Salzpartikeln: ein Teil des Salzes weist eine wasserlösliche, retardierend wirkende Schicht auf seiner Oberfläche auf und der andere Teil enthält Füllstoffe und Pigmente. Dadurch wird zunächst der zweite Teil mit einem Farbeffekt und nach einigen Minuten der erste Teil gelöst, welcher den eigentlichen Badezusatz mit Aromen, Tensiden zeitverzögert freisetzt.

In der DE 202012005506 U1 werden feste Badezusätze z.B. auf Salzbasis beschrieben, welche 2 aufeinander aufbauende körnige Wirkphasen mit sichtbarem und auch olfaktorisch wahrnehmbaren Einsetzen der zweiten Wirkphase durch Änderung der Farbe und des Duftes umfasst. Hierbei beträgt das Mengenverhältnis der Trägerpartikel der ersten Phase zu den Trägerartikeln der zweiten Phase ca. 4 :1 bis 1,2:1. Auch hier ist eine Retardierungsüberzugsschicht wie vor allem aus Zucker wie Glucose, Lactose, Saccharose, Gelatine geeigneter Art oder Schellack auf der zweiten Phase erforderlich.

Die oben genannten Zubereitungen erfordern jedoch jeweils besondere galenische Transportmechanismen oder Herstellungsmaßnahmen, insbesondere die Bildung von Umhüllungsschichten. Von Vorteil wären jedoch auch Produkte, bei welchen derartige besondere Ausgestaltungen nicht erforderlich sind und wobei dennoch ein gewünschter, insbesondere zeitlich differenzierter Farbstoffaustrag erfolgt.

### Aufgabe der Erfindung

Aufgabe vorliegender Erfindung ist es daher, einen Badezusatz bereitzustellen, der ohne besondere galenische Maßnahmen wie spezielle Transportmechanismen oder den Einsatz von separierten bzw. separierenden umhüllenden Retardierungsschichten gut herstellbar ist und bei dem physiologische Wirkungen, insbesondere Farbwechsel während der Anwendung zeitlich differenziert und klar abgegrenzt voneinander stattfinden sollen. Ferner sollen keine sichtbaren Rückstände auf dem Badewasser zurückbleiben.

### Lösung der Aufgabe

Diese Aufgabe wird erfindungsgemäß gelöst durch die Bereitstellung eines festen, 2-Komponenten-Kombinations-Badezusatzes auf Basis eines wasserlöslichen Trägers, vor allem auf Salzträgerbasis, mit zwei unterschiedlichen Formkörpern 1 und 2, nämlich der Formkörperkomponente 1 mit loser, schüttbarer Matrix und der Formkörperkomponente 2 mit gepresster Matrix. Dabei umfasst die Formkörperkomponente 1 mit loser Matrix neben dem Träger 1, insbesondere Meersalzträger, eine Wirkstoffgruppe 1, umfassend einen oder mehrere Farbstoffe und wahlweise einen oder mehrere geeignete Zusatzstoffe, ausgewählt aus Emulgatortensiden, Zusatzwirkstoffen wie z.B. Aromen, einem oder mehreren Lipiden, galenischen Zusatzstoffen oder Mischungen hiervon. Die zweite Formkörperkomponente 2 mit gepresster Matrix und einem Träger 2 auf Salzbasis umfasst eine Wirkstoffgruppe 2 mit einem oder mehreren Lipiden, insbesondere einem oder mehreren flüssigen Ölen, sowie einem oder mehreren, von in der Formkörperkomponente 1 vorhandenen Farbstoffen verschiedenen Farbstoffen sowie ggf. Zusatzstoffe, ausgewählt aus Zusatzwirkstoffen wie Aromen, galenischen Zusatzstoffen. Dabei ist mindestens ein Lipid in der Komponente 2 ein flüssiges Öl. Erfindungsgemäße Kombinationsbadezusätze umfassen bzw. bestehen aus Formkörperkomponente 1 und Formkörperkomponente 2 insbesondere im Verhältnis (Gewichtsverhältnis) 5:1 bis 1:1.

Erfindungsgemäß bedeutet 2- Komponenten-Kombinations-Badezusatz einen festen Badezusatz, welcher 2 Formkörper mit unterschiedlich beladenen und unterschiedlich physikalisch gestalteten festen Trägerpartikeln, insbesondere Salzpartikeln, mit unterschiedlicher Matrix (lose, schüttbar: Formkörperkomponente 1 bzw. fest, gepresst: Formkörperkomponente 2) aufweist.

Beide Komponenten 1, 2 der Badezusatz-Kombination werden gleichzeitig in die Badeflotte eingetragen. Dabei löst sich aufgrund der sowohl substantiell als auch physikalisch unterschiedlich beschaffenen Matrices zunächst die erste kristalline Formköperkomponente 1 mit den dort vorhandenen Träger- sowie Wirkstoffen wie vor allem Farbstoffen, ggf. Tensiden. Nach einigen Minuten löst sich die zweite Formkörperkomponente 2 mit den dort vorhandenen Träger- und insbesondere unterschiedlichen Farbstoff(en). Damit ist es möglich, einen klaren Farbwechsel in der Badeflotte zu bewirken. Darüber hinaus bleiben keine Rückstände auf der Badewasseroberfläche, wie dies bei Anwesenheit von Retardierungsmitteln der Fall sein kann. Bevorzugt wird diese visuelle Wirkungs- (Farb-)änderung auch geruchlich (aromaspezifisch) erkennbar, indem mit dem Farbstoffstoffwechsel durch die zusätzliche Anwesenheit von Zusatzwirkstoffen in einem oder beiden Formkörperkomponenten wie Aromen (ätherische Öle/Extrakte) gleichzeitig eine Aromainduktion, vorzugsweise ein Farbwechsel und ein Geruchs-(Aroma-)wechsel stattfindet. Zusätzlich kann neben der Reinigung (Emulgatortensid) gleichzeitig ein pflegender Ausgleich des Lipidverlusts der Haut (durch das oder die Lipide, insbesondere auch durch das flüssige Öl in der zweiten Phase) wirksam erfolgen.

Mit den erfindungsgemäßen festen Kombinations-Badezusätzen kann somit auf aufwendige Herstellungstechniken verzichtet werden, da die zeitverzögerte Wirkstofffreisetzung durch den Einsatz verschiedener Matrixformen einfach erzielbar ist. Dabei kann eine Zeitsteuerung insbesondere durch den Gehalt an flüssigem Öl, auch durch den Grad der Pressung des Formkörpers 2 bewirkt werden. Durch die Festigung, vor allem Pressung des Formkörpers 2, insbesondere zu Tabletten mit glatter Oberfläche, kann eine stärkere Löslichkeitsverzögerung der Wirk- insbesondere Farbstoffe und ggf. Zusatz(wirk)stoffe erzielt werden.

Es war überraschend, dass ein klar getrennter Farb- bzw. ggf. Aromawechseleffekt stattfindet, da hier anders als im Stand der Technik keine besonderen umhüllenden Retardierungsmittel eingesetzt werden und nur aufgrund der unterschiedlichen Matrixbeschaffenheit bzw. der Anwesenheit eines flüssigen Öls in einer festen Matrix eine un-differenzierbare Vermengung der Farbstoffe vermieden werden kann. Dies gilt um so mehr, als dass davon ausgegangen werden musste, dass die in der Badeflotte herrschenden Temperaturen eher eine Angleichung als eine Differenzierung der Löslichkeiten beider Komponenten bedingen, insbesondere auch dann, wenn das Substanzgemisch im schnelllöslichen Formkörper 1 Lipide umfassen kann, zumal der oberflächliche Substanzauftrag sowohl im langsamer löslichen Formkörper 2 als auch im Formkörper 1 durch Sprühung erfolgen kann, und das oberflächliche Auftragen durch z. B. Besprühen im allgemeinen bei der Herstellung retardierender Umhüllungsschichten eingesetzt wird.

### Nähere Erläuterung der Erfindung

Die Erfindung betrifft feste 2- Komponenten-Kombinations-Badezusätze aus 2 Formkörperkomponenten 1, 2 mit unterschiedlichen Matrices. Jede der beiden Komponenten umfasst und vorzugsweise besteht aus einem oder mehreren wasserlöslichen Trägern 1, 2 in einer Menge von je mehr als 70 Gew.%, vor allem bis zu 99,9 Gew.%, insbesondere von 87,5 bis 99,7 Gew. % oder 95 bis 99,6, insbesondere bis 98, vor allem bis 97,5 Gew.%, insbesondere Salzträger. Vorzugsweise umfasst die Komponente 1 96 bis 99,7 Gew.% Salzträger und die Komponente 2 96 bis 98 Gew.% Salzträger. Als Rest sind in den Komponenten 1, 2 vor allem 0,1 bis 12,5 Gew.%, insbesondere 1 bis 5 Gew. % der Wirkstoffgruppe 1 bzw. 2 mit 0,05 bis 3, vorzugsweise 0,1 bis 3 Gew. % Farbstoffen (Komponente 1, 2), 0,1 bis 2,5 % einem oder mehrerer Lipide, insbesondere flüssigen Ölen (Komponente 2) und 0,5 bis 7 Gew.% Zusatzstoffen, ausgewählt aus galenischen Zusatzstoffen (Konditioniermitteln), Zusatzwirkstoffen, mit der Maßgabe, dass die 2. Komponente ein gepresster Formkörper 2 vorzugsweise auf Salzträgerbasis, mit Substanzen der Wirkstoffgruppe 2 (Farbstoffe, Lipide, insbesondere flüssige Öle, Zusatzstoffe) ist, während die erste Komponente 1 eine loser, nicht gepresster, schüttbarer Formkörper 1 auf Basis eines wasserlöslichen Trägers 1, insbesondere Salzträgers, mit Substanzen der Wirkstoffgruppe 1 (Farbstoffe, Zusatzstoffe wie beschrieben z.B. Vitamine, flüssige und/oder feste Lipide oder Mischungen hiervon) ist, und mit der Maßgabe, dass mindestens ein flüssiges Lipid, insbesondere ein flüssiges Öl vorhanden ist. Der/die Farbstoffe der Komponente 1 ist/sind verschieden von dem/den Farbstoff(en) der Komponente 2. Zusatzwirkstoffe können ausgewählt werden aus ätherischen Ölen, Parfümstoffen, hautwirksamen Substanzen wie (Pflege-)Lipiden, Vitaminen. In Komponente 1 sind vorzugsweise Vitamine wie Vitamin E, und/oder Lipide (flüssig, fest oder Mischungen hiervon) vorhanden, in Komponente 2 sind vorzugsweise Konditioniermittel wie Lactose und/oder Silica vorhanden.

Die Mengen der einzelnen Inhaltsstoffe sind so beschaffen, dass sich jeweils 100 Gew.% ergeben.

Bevorzugt umfasst oder insbesondere besteht die Formkörperkomponente 1 mit loser Matrix aus 90 bis 99,5 Gew.% des wasserlöslichen Trägers und 0,5 bis 10 Gew.% der Wirkstoffgruppe 1, und die Formkörperkomponente 2 mit gepresster Matrix, aus 90 bis 97 Gew.% des wasserlöslichen Trägers 2 und 10 bis 3 Gew.% der Wirkstoffgruppe 2.

Bevorzugt ist die Wirkstoffgruppe 1 auf den Träger der Formkörperkomponente 1 mit loser Matrix 1 oberflächlich aufgetragen, insbesondere aufgesprüht und die Wirkstoffgruppe 2 nach dem Auftrag auf die Partikel wie z.B. durch Sprühung in den Träger der Formkörperkomponente 2 mit fester, gepresster Matrix überwiegend eingepresst.

Die Wirkstoffgruppe 1 umfasst und bevorzugt besteht aus einem oder mehreren Farbstoff-(en) und einem oder mehreren Zusatzstoffen, ausgewählt aus Emulgatortensiden, insbesondere anionischen Succinaten, anionischen Sulfaten, anionischen Tauraten und einem oder mehreren Zusatzwirkstoffen, vor allem einem oder mehreren Aromen, Vitaminen, Pflegewirkstoffen wie Pflegelipide oder Mischungen hiervon.

Die Wirkstoffgruppe 2 umfasst und bevorzugt besteht aus einem oder mehreren Lipiden, vor allem einem oder mehreren flüssigen Ölen, einem oder mehreren Farbstoffen, der/die verschieden ist/sind von dem/den Farbstoffen der ersten Wirkstoffgruppe 1 sowie ggf. einem oder mehreren Zusatzwirkstoffen wie Aromen, Vitaminen, und einem oder mehreren galenischen Hilfsstoffen, vor allem Konditioniermitteln, ausgewählt aus Silica, Kohlenhydrate.

### Bevorzugte Ausführungsformen

Bevorzugte Ausführungsformen für die Formkörperkomponente 1 sind folgende: Ausführungsform 1.1.

Diese lose, schüttbare kristalline oder pulverförmige, vor allem grobkristalline Formkörperkomponente 1 mit loser Matrix umfasst/und insbesondere besteht aus/neben dem Träger 1, insbesondere dem Salzträger 1, die/und der Wirkstoffgruppe 1 mit einem oder mehreren Farbstoffen, sowie Zusatzstoffe, ausgewählt aus einem oder mehreren Reinigungstensiden, (Emulgatortensid), einem oder mehreren Zusatzwirkstoffen, ausgewählt aus einem oder mehreren ätherischen Ölen (Aromen), einem oder mehreren festen oder flüssigen Lipiden oder Mischungen hiervon, oder Mischungen derartiger Zusatzstoffe.

### Ausführungsform 1.2.

Eine andere Ausführungsform 1.2 betrifft Formkörperkomponenten 1.2, worin neben dem Träger 1 in der Wirkstoffgruppe 1 ein oder mehrere Farbstoffe, insbesondere Gelb- und/oder Rot-Farbstoffe, ein flüssiges und ein festes Lipid, sowie ein oder mehrere Emulgatortenside und/oder Aromen, insbesondere ätherische Öle wie z.B. erfrischende, belebende ätherische Öle und/oder Parfümöle vorhanden sind.

### Bevorzugte Ausführungsformen für die Formkörperkomponente 2 sind folgende: Ausführungsform 2.1

Diese betrifft feste, vor allem gepresste, insbesondere tablettenförmige Formkörperkomponenten 2 mit fester Matrix, worin neben/bzw. welche aus dem Salzträger 2 die Wirkstoffgruppe 2 mit einem oder mehreren Farbstoffen, einem oder mehreren flüssigen Ölen, insbesondere Distelöl, und einem oder mehreren Konditioniermitteln, insbesondere Silica, Kohlenhydrate wie Lactose, Stärke, ggf. Restwasser, oder Mischungen hiervon, umfasst sind bzw. daraus bestehen.

### Ausführungsform 2.2

In einer anderen Ausführungsform 2.2. umfasst bzw. besteht der Formkörper 2 den/bzw. aus dem Salzträger 2 und der Wirkstoffgruppe 2 aus Farbstoffen wie Orange, Rot, Violett, ein oder mehreren flüssiges/n Ölen, Aromen, Silica, Lactose.

In den oben beschriebenen Ausführungsformen können anstelle oder zusätzlich zu den Aromastoffen auch Vitamine vorhanden sein. Die Aromastoffe können auch nur in einer Komponente vorhanden sein, während jedoch mindestens ein Farbstoff in jeder Komponente 1, 2 enthalten ist, der jeweils verschieden von dem Farbstoff in der anderen Komponente ist. Bevorzugt sind unterschiedliche Farbstoffe und gleiche/oder unterschiedliche Aromen und/oder Vitamine in beiden Komponenten vorhanden. Insbesondere sind sowohl Farbstoffe als auch Zusatzwirkstoffe in jeweils beiden Formkörperkomponenten umfasst, welche jeweils verschieden voneinander sind. Erfindungsgemäße 2-Komponenten-Badezubereitungen können daher vorzugsweise aus dem/den Trägermaterialien 1, 2 (vorzugsweise Salz für Komponente 1 und 2), dem/den Farbstoffen (Komponente 1 und 2), dem/den Zusatzwirkstoffen (insbesondere Vitamine und/ oder Lipide (fest und/oder flüssig wie nachfolgend beschrieben) in Komponente 1), dem/den flüssigen Öl(en) (Komponente 2) bzw. Emulgatortensid(en) (Komponente 1) und dem/n genannten galenischen Zusatzstoff(en) (Komponente 1, 2), insbesondere Konditioniermitteln in Komponente 2 bestehen.

Bevorzugte Kombinationsbadezusätze umfassen bzw. bestehen aus Formkörperkomponente 1.1 und Formkörperkomponente 2.1, insbesondere im Verhältnis (Gewichtsverhältnis) 4:1 bis 2:1.

Eine weitere Ausführungsform betrifft Kombinations-Badezusätze, welche umfassen bzw. bestehen aus:
1:1 bis 5:1, vorzugsweise 2:1 bis 4:1 Teile loser Formkörper 1 zu gepresstem Formkörper 2 mit:
   einem Formkörper 1 aus 97 bis 99,7 Gew.% eines festen wasserlöslichen Trägers,
   ausgewählt aus Salzen, Harnstoff, Kollagenhydrolysat, Cellulose, Kohlenhydraten wie Saccharose, Fructose, Dextrose, Lactose, 0,05 bis 0,9 Gew.% Farbstoff, 0,1 bis 0,5 Gew.% Emulgatortensid, 0,1 bis 1,1 Gew.% Zusatzstoffe, ausgewählt aus Wirkstoffen, insbesondere Aromen, Lipiden, z. B. 0,1 bis 1 Gew.% (Pflegewirkstoffen);
   und mit einem gepressten Formköper 2 aus 96 bis 98,85 Gew.% Salz, insbesondere Meersalz, und 0, 05 bis 0,9 Gew.% Farbstoff, der verschieden ist von dem/den Farbstoffen der Komponente 1, sowie 0,5 bis 1,5 Gew.% flüssige(s) Öl(e) und 0,6 bis 1,6 Gew. % Zusatzstoffe, ausgewählt aus Konditioniermitteln, Wirkstoffen, insbesondere Aromen.

Besonders bevorzugt sind Kombinationsbadezusätze in einer Gesamtmenge von 60-80 g (z. B. als Einmaldosis) aus 40 bis 65 g der losen Formkörperkomponente 1 und 20 bis 15 g der gepressten Formkörperkomponente 2.

Als Trägerpartikel für den Formkörper 2 und bevorzugt auch für den Formkörper 1 werden vor allem Solesalze, Totes Meersalz, Siedesalz, Meersalz, insbesondere natürliches Meersalz, Mineralsalz (wie Natriumchlorid, Natriumsulfat) oder Mischungen hiervon eingesetzt.

Andere Trägermaterialien sind z. B. ausgewählt aus Zuckern, Stärke, insbesondere Kartoffelstärke oder auch Harnstoff oder Mischungen derart, dass in der Komponente 2 als Träger ein Salz und in der anderen Komponente 1 als Träger ein Salz oder ein anderer wasserlöslicher Träger wie Cellulose, Harnstoff vorhanden ist.

Vor allem geeignet sind Solesalz, Meersalz, Mineralsalz oder Mischungen hiervon.

Insbesondere bevorzugt ist der Träger beider Formkörper ein oder mehrere Salze, wobei mindestens in einem Formkörper, bevorzugt im Formkörper 2, Meersalz vorhanden ist.

Insbesondere werden als Träger in der Komponente 1 Salze wie vor allem Steinsalz, Meersalz, Kombinationen hiervon gewählt.

Die Formkörperkomponente 1 mit loser Matrix 1 liegt insbesondere als Granulat (grobkristallin) oder als Pulver (feinkristallin) vor. Die Formkörperkomponente 2 mit fester, vor allem gepresster Matrix liegt bevorzugt als scheibenförmige Tablette oder zylinderförmiges Baderöhrchen, insbesondere als Tablette mit glatt gepresster Oberfläche vor. Bei der bevorzugten Ausführungsform Tablette beträgt der Durchmesser insbesondere 3 - 6 mm.

Bevorzugt wird für den Formkörper 1 als erste Trägerkomponente ein grobkristalliner Träger, vorzugsweise mit einer mittleren Korngröße von 1 -4 mm, und für den zweiten Formkörper 2 als Trägerkomponente ein feinkristalliner Träger der mittleren Korngröße 0,2 - 0,8 mm eingesetzt.

Als Reinigungsmitteltenside (Emulgatortenside) in der ersten Komponente werden vor allem wasserlösliche anionische Emulgatortenside wie Sulfosuccinate, Alkyl- und Alkylether-Sulfate und -Taurate wie nachfolgend beschrieben eingesetzt.

Als Lipide, insbesondere flüssige Öle für beide Komponenten 1 2, vor allem in Komponente 2, eignen sich vor allem natürliche flüssige Öle, insbesondere Distelöl, oder Sonnenblumen-, Sojaöl, Olivenöl, Pfirsichkern-, Aprikosenkern-, Traubenkern-, Erdnußöl, Mandel-, Nerz-, Weizenkeim-, Sesamöl, Avocadoöl, Palmöl, Pinienkernöl, Reiskeimöl, Macadamianussöl, Palmkernöl, Papayaöl.

Als Farbstoffe für beide Formkörper können vor allem Indigo, Brillantblau, Cochenillerot, Brilliantgrün, Hellgelb, Karminrot, Patentblau, Ponceau, Gelborange E110, Amaranth, Brillantrosa, Echtgelb, Carotinoide, Rote Beete Extrakt, Chlorophyllin, oder auch Gelb (Riboflavin), Chinolingelb oder Mischungen gewählt werden mit der Maßgabe, dass ein Farbstoff der Komponente 1 verschieden von einem Farbstoff der Komponente 2 sein muss.

In einer besonderen Ausführungsform ist der Farbstoff der ersten Komponente ausgewählt aus Gelb (z.B. Chinolingelb E 104), und der Farbstoff der zweiten Komponente ausgewählt aus Cochenille Rot (Ponceau 4R/Cochenille rot).

Alternativ kann der Farbstoff der ersten Formkörperkomponente 1 ausgewählt sein aus Cochenille Rot (Ponceau 4R/Cochenille rot) und der Farbstoff der zweiten Formkörperkomponente 2 ausgewählt sein aus Brillantblau.

Als Zusatzstoffe können Zusatzwirkstoffe für beide Komponenten insbesondere ausgewählt werden aus Vitaminen, ätherischen Ölen/Extrakten aus Pflanzen, oder Kombinationen hiervon wie nachfolgend beschrieben. Dabei können der oder die Zusatzwirkstoffe der ersten Komponente gleich oder verschieden jenem/n der zweiten Komponente sein. Bevorzugt liegen Kombinationen, insbesondere unterschiedliche Kombinationen von Zusatzwirkstoffen in den beiden Komponenten vor. Insbesondere bevorzugt ist mindestens ein Wirkstoff der zweiten Komponente nicht gleich mit einem Wirkstoff der ersten Komponente, und ist gewählt aus Aromen aus der Gruppe, umfassend oder insbesondere bestehend aus Minzöl, Rosmarinöl, Zitronenöl, Thymianöl, Salbeiöl, Orangenöl, Passionsblumen-Extrakt, Johanniskraut Extrakt, Lavendelöl, Melissenöl, Wacholderöl, Wintergrünöl, Eukalyptusöl, Korianderöl, Ingwerextrakt, Beinwell-Extrakt, Arnika-Extrakt, Kiefernnadelöl, Fichtennadelöl, Ylang Ylang Öl, Limetten-öl, Mandarinenöl, Grapefruitöl oder Mischungen hiervon.

Weitere Wirkstoffe sind Vitamine wie Vitamin E, C oder und geeignete Derivate hiervon wie nachfolgend beschrieben.

Die in den erfindungsgemäßen Kombinationen eingesetzten Lipide können auch als hautpflegende Wirkstoffe agieren. Dazu gehören vor allem feste Lipide, z.B. pulverisierte Kakaobutter, oder Kombinationen fester Lipide mit flüssigen Lipiden wie sie vorzugsweise in Komponente 1 eingesetzt werden, wie Ölen, z.B. feste Kakaobutter + Macadamianussöl, im Verhältnis von 1:10 bzw. 1:2. In der Komponente 1 können feste Lipide allein, in der Komponente 2 werden vor allem nur flüssige Lipide eingesetzt. Als galenische Zusatzstoffe, insbesondere im Formkörper 2, können vor allem Konditioniermittel wie Adsorber, z.B. Silica, Kohlenhydrate eingesetzt werden.

Je nach ausgewählten Farb-bzw. Farb-und (Aroma-)Wirkstoffen kann somit ein besonderes Farbenspiel wie gelb-orange-rotes Sonnenpanorama, türkis-blaue Meereslagune, gelb-grüne Waldlandschaft, Brillantrosa-Lavendel-Landschaft z.B. zur Erlangung eines entspannenden, beruhigenden, anregenden Effekts erreicht werden. Neben der durch die in den Formkörpern 1, 2 inkorporierten Lipide, insbesondere Öle, zusätzlich erzielbaren Pflege können durch Inkorporierung geeigneter Wirkstoffe wie Aromen (ätherische Öle) auch entsprechende physiologische Wirkungen angesprochen werden wie z.B. (Haut-)Beruhigung, Entspannung, Muskelentspannung, Belebung, Durchblutung, z.B. mittels ätherischer Öle/Extrakte aus Orange, Minze, Thymian, Salbei, Eukalyptus bzw. Lavendel (Formkörper 1, 2), z. B. in Kombination mit Olivenöl, Macadamianussöl, Distelöl, (Formkörper 2). Dabei kann der Farbstoff mit dem/den Aromen korreliert werden wie gelb/grüne Färbung + Minz-/Eukalyptusöl, rosa Färbung + Lavendelaroma, orange-rot-Färbung + Orangen-/Grapefruitextrakt, blaue Färbung + Salbei.

### Nähere Beschreibung der Inhaltsstoffe

### 1. Farbstoffe (in Komponente 1 und 2)

Die Farbstoffe werden ausgewählt aus den hierfür dem Fachmann bekannten Substanzen, insbesondere natürlichen Farbstoffen. Sie werden vor allem entsprechend der gewünschten Wirkung ausgewählt. So kann für einen beruhigen/lindernden Effekt zunächst die Farbe Gelb (Riboflawin-5-phosphat) in der ersten Komponente und die Farbe Orange oder Blau (z. B. Indigo, Patentblau, Brillantblau) in der zweiten Komponente gewählt werden, was einen zeitverzögerten Farbumschlag nach Gelborange bzw. Grün bewirkt. Alternativ kann in der ersten Komponente ein Rotton (Cochenillerot, Karminrot) gewählt werden, der mit einem Blauton (z. B. Patentblau) in der zweiten Komponente zu einem Violettton führt. Eine Kombination mit geeigneten Aromen kann zusätzlich zu einem physiologischen Anregungs-/Durchblutungseffekt führen. Der Fachmann kann daher geeignete Kombinationen auswählen, je nach beabsichtigtem Effekt in der Komponente 1 bzw. 2 oder in Bezug auf die Gesamtwirkung. Bevorzugt für den Farbumschlag sind die Formkörper 1- Farben Gelb, Orange, Rot, Grün, Rosa und die Formkörper 2- Farben Rot, Blau, Violett. Der Farbumschlag kann auch mit Misch-Farben oder Abstufungen der Farben realisiert werden.

Zu den geeigneten Farbstoffen gehören z.B.:
Chinolingelb, Gelb (Riboflavin,-5-Phosphat-Natrium)Chlorophyll, Hellgelb (z.B. Sudan), Echtgelb, Gelborange (E 110), Rot 10B, Rote Beete, Cochenillerot, Indigoblau, Brillant-blau, Holunderextrakt (Anthocyane), Carotinoide, Vitamin E, , Brillantrosa, Amaranth, Ponceau, Karminrot, Brilliantgrün, Patent- Blau.
Besonders bevorzugt sind: Gelb wie oben genannt, Gelborange (insbesondere Formkörperkomponente 1), oder Rot (z.B. Cochenille), letztere vor allem im Formköperkomponente 2.
Besonders bevorzugt werden in der Trägerkomponente 1 die Farben Gelb und Rot (z.B. Cochenille) und in der Trägerkomponente 2 Rot (z.B. Cochenille), Grün, Blau (z. B. Brillantblau oder Patentblau).

Die genannten Farbstoffe sind insbesondere natürlichen Ursprungs und ungiftig bzw. auch in der Lebensmittelindustrie zulässig.

### 2. Feste und flüssige Lipide in Komponente 1 und/oder 2

Feste und flüssige Lipide können in der Formkörperkomponente 1 und 2 eingesetzt werden mit der Maßgabe, dass als Lipid in Komponente 2 mindestens ein flüssiges Lipid, insbesondere ein flüssiges Öl, vorhanden ist. In Komponente 1 können Lipide als Zusatzwirkstoffe dort vorhanden sein.

Erfindungsgemäße Lipide umfassen vor allem die nachfolgend genannten Substanzgruppen, nicht jedoch ätherische Öle/Extrakte, Parfümöle.

Die flüssigen Öle können vor allem aus flüssigen natürlichen Ölen gewählt werden. Diese werden insgesamt in einer geringen Mengen von 0,1 bis 2 Gew.%, insbesondere 0,1 bis 1,5 Gew.%, bezogen auf die Gesamtzubereitung der Komponente 1 und in einer geringen Mengen von 0,1 bis 2 Gew. %, insbesondere 0,5 bis 1,5 Gew.%, bezogen auf die Gesamtzubereitung der Komponente 2 eingesetzt. Sie werden vorzugsweise aus-gewählt aus Distelöl, Reiskeimöl, Olivenöl, Macademianussöl, Walnussöl, Palmöl, Sonnenblumenöl, Sojaöl, Pfirsichkernöl, Aprikosenkernöl, Traubenkernöl, Ricinusöl, Erdnußöl, Mandelöl, Nerzöl, Weizenkeimöl, Sesamöl, Palmkernöl, Nachtkerzenöl, Avocadoöl, oder Illipé Butter, Haselnussöl, Hagebuttenkernöl, Baumwollsamenöl, Papayaöl oder anderen in diesem Zusammenhang bekannten Ölen, oder Mischungen hiervon.

Besonders bevorzugte flüssige Öle sind Reiskeimöl, Distelöl, Mandelöl, Sojaöl und Macadamianussöl, insbesondere für Komponente 2.

Feste Lipide, wie sie insbesondere in Komponente 1 (Zusatzwirkstoffe) eingesetzt werden können, werden vor allem ausgewählt aus Wachsen (enthaltend Ester langkettiger Alkohole mit langkettigen Fettsäuren), festen Fetten (Butter, enthaltend u.a. Glyceride langkettiger Fettsäuren u.a.) wie insbesondere Jojobaöl (flüssiges Wachs), Bienenwachs, Canaubawachs, Kakaobutter, insbesondere pulverförmige Kakaobutter, Mangobutter, oder auch Sheabutter. Feste Lipide sind insbesondere in Komponente 1 enthalten.

Derartige Lipide können insbesondere hautwirksam (z. B. rückfettend und feuchtigkeitsbewahrend) wirken, vor allem, wenn Emulgatortenside eingesetzt werden.

### 3. Zusatzstoffe

### 3.1 Zusatzwirkstoffe (in Komponente 1 und/oder 2)

Diese können vor allem ausgewählt werden aus ätherischen Ölen/Extrakten bzw. Pflanzenextrakten, Vitaminen, Hautwirksamen Substanzen wie Pflegestoffen, Parfümstoffen.

### 3.1.1 Ätherische Öle/Extrakte bzw. Pflanzenextrakte

Diese können gewählt werden aus Ölen /Extrakten aus:
Orange, Minze, Mango, Pfefferminze, Rosmarin, Melisse, Zitrone, Lavendel, Kiefernnadel, Wacholder, Zypresse, Feigen, Zedernholz, Lotus, Kamille, Ylang Ylang, Wasserlilie, Passionsblume, Korianderöl, Thymian, Limetten, Grapefruit, Calendula, Johanniskraut, Mandarine, Wacholder, Baldrian, Zitronenmelisse, Lavendel, Eukalyptus, Lemongras, Fichtennadel, Salbei, Lindenblüten, Teebaumöl, Wintergrün, Rosenholz, Palmarosa, Cranberry, Granatapfel, Ananas, Guave, Echinacea, Efeublätterextrakt, Heidelbeerextrakt, Kiefernnadelöl, Fichtennadelextrakt, Kaki, Melonen, Magnolien-, Ananas-Extrakt, Grapefruitöl u. ä. oder Mischungen hiervon wie Mischungen aus Minzöl, Rosmarin, Melisse, Arnika-Extrakt, Calendula äth., Beinwell-Extrakt,
Orangenöl und Minze, Mandarine und Limetten, Johanniskraut und Palmarosa, Fichtennadel und Teebaumöl u.a.

Insbesondere bevorzugt sind: Orangenöl, Minzöl, Rosmarinöl, Zitronenöl, Lavendelöl, Melissenöl oder auch Thymianöl, Salbeiöl, Passionsblumenextrakt, Johanniskraut-Extrakt, oder Mischungen hiervon.

Als Zusatzwirkstoffe, insbesondere ätherische Öle der ersten FormkörperKomponente 1 können vor allem solche gewählt werden, die eine bestimmte physiologische Erstwirkung bedingen sollen wie Durchblutung, Entspannung, Regenerierung, auch der Haut, Linderung von Beschwerden des Bewegungs-und/oder Atmungsapparates.

Folgende Korrelationen von Farbstoffen und ätherischen Ölen sind möglich:
Die Zusatzwirkstoffe, insbesondere ätherischen Öle/Extrakte, der Formkörperkomponente 2 sind vor allem ausgewählt zur Herbeiführung einer deutlichen Differenzierung des Farbeffektes aus der ersten Formkörperkomponente, ggf. auch zur Verstärkung einer Aromawirkung und/oder Bewirkung eines zusätzlichen Effekts der Aromawirkung aus der ersten Formkörperkomponente (wie beispielsweise Entspannung, Muskelrelaxierung, Erkältungslinderung, bei Stress, Verspannung, Unruhe). Besonders geeignet sind hier Lavendelöl, Melissenöl, Lemongrasöl, Speik-Lavendelöl, Baldrianöl, Rosmarinöl oder Kombinationen hiervon.

### 3.1.2. Vitamine (in Komponente 1 und/oder 2)

Gegebenenfalls können als Zusatzwirkstoffe auch geeignete Mengen an Vitaminen, insbesondere Haut-wirksamer Vitamine eingesetzt werden. Hierzu gehören beispielsweise Vitamin C (-Palmitat, Calciumascorbat/Natriumascorbat, Kaliumascorbat); Vitamine der B- Gruppe wie Thiamin (B1), z. B. Thiaminnitrat, Riboflavin, Niacin/Niacinamid (B3), Pantothensäure (B5) wie Calciumpantothenat, Vitamin H (B7), Biotin oder auch Carotinoide wie Beta/- Alpha-Carotin, Beta-Cryptoxanthin, Lutein, Zeaxanthin, Lycopin.

Geeignet sind auch Carotinoide, die gleichzeitig auch als Farbstoffe eingesetzt werden.

Besonders geeignet sind auch Vitamin E (Tocopherol, Tocopherylacetat, Tocopherylpalmitat; Tocopherylsuccinat), Vitamin A (Retinol, Retinylpalmitat, Retinylacetat, Retinylproprionat).

Es kann auch von Vorteil sein, Vitaminkombinationen einzusetzen, je nach gewünschter oder besonderer Indikation.

### 3.1.3. Hautwirksame Zusatzwirkstoffe (in Komponente 1 und/oder 2)

Geeignete, auch hautwirksame Wirkstoffe können gewählt werden aus essentiellen ungesättigten Fettsäuren und deren Estern wie Linol- oder Linolensäure, Glyceryl Linoleate, Glyceryl Linolenate, oder auch aus durchblutungsfördernden Mitteln wie Alpha- und Betahydroxysäuren und deren Derivate, z. B. Glykol-, Äpfel-, Zitronen-, Wein-, Milchsäure, Salicylsäure, Isopropylbenzylsalicylate, C12-13 Alkyl Lactate, Nikotinsäurederivate wie Methyl- oder Tocopherylnikotinat, oder auch aus der Gruppe, umfassend antiphlogistische und antibakterielle Substanzen wie Triterpene ( z. B. Ursolsäure, Glycyrrhizinsäure oder Glycyrrhetinsäure und deren Derivate, z. B. Stearyl Glycyrrhetinate, Kaliumglycyrrhinat); oder Pantothensäurederivate, z. B. D- Panthenol, Panthenyltriacetat; oder auch aus Polyphenolen, Flavonoiden (z. B. Ferulasäure und deren Ester, Rutin, oder Isoflavonen wie Soja-Isoflavon oder Coenzym Q 10).

Sofern erforderlich oder gewünscht können auch Feuchthaltemittel wie Glycerin, Propylenglykol oder Polyethylenglykole, Polypropylenglykol, Butylenglykol, Sorbitol, Allantoin, Dexpanthenol oder Polymere, z.B. Polyquaternium-Typen, Kollagen oder dessen Hydrolysate, Aminosäuren eingesetzt werden.

Wie oben erläutert, können hautwirksame Wirkstoffe, vor allem hautpflegende Stoffe, auch solche aus der Gruppe der Lipide, insbesondere flüssige ungesättigte Öle sein. Vesikelbildende Lipide können, müssen aber nicht vorhanden sein. Dazu gehören vor allem Phospholipide, wie Lecithin (z.B. Ei- oder Soja-Lecithin), oder Phosphatidylcholin, oder Phosphatidyldiethanolamin, Phosphatidylserin, Phospatidylinosit, z. B. aus Soja, Raps, Baumwollesamen, Ei, sowie deren Mischungen. Weitere geeignete Komponenten zur Bildung von Vesikeln sind Sphingolipide (z.B. Ceramide, Cerebroside, Sphingosin, Sphingomyelin), oder auch ethoxylierte Sterole wie Phytosterole (Gemische aus Sistosterol, Camposterol und Stigmasterol), z.B. Ethoxylate hiervon wie PEG-5-Sojabean Sterol oder PEG-5 Rapeseed Sterol = Generol® 122 E5 bzw. Generol® R E5.

### 3.1.4 Parfümstoffe (Zusatzwirkstoffe) in Komponente 1 und/oder 2

Zusätzlich oder alternativ zu vorgenannten Aromen können als Zusatzwirkstoffe auch dem Fachmann hierfür bekannte Parfümstoffe, insbesondere natürlichen Ursprungs, hinzugesetzt werden, deren Art/Mischung auf die zu erzielende Wirkung abgestimmt ist.

Bevorzugte Zusatzwirkstoffe sind ausgewählt aus Vitaminen (Komponente 1, 2), oder aus Vitaminen in Kombination mit einem oder mehreren wasserlöslichen und wasserunlöslichen, auch als Haut-Pflegestoffe wirksamen Lipiden (Komponente 1). Letztere sind vor allem gewählt aus festen Lipiden in geringen Mengen wie Kakaobutter, vorzugsweise pulverisiert. Das oder die Vitamine, das oder die Lipide oder Kombinationen hiervon können insbesondere auch in Mischung mit einem oder mehreren ätherischen Ölen/Extrakten und/oder Parfümstoffen, insbesondere pflanzlichen Ursprungs, wie oben beschrieben, vorliegen. Besonders geeignet sind Kombinationen aus Vitamin E + Parfümöl, Vitamin E + Kakaobutter pulverisiert, und auch Vitamin E + Calendula-Extrakt. Üblicherweise kann insofern auf synthetische Wirkstoffe bzw. Inhaltsstoffe weitestgehend verzichtet werden. Als Parfümstoffe können wie erwähnt auch Aromen (ätherische Öle/Extrakte) verwendet werden. Es werden ferner keine Konservierungsmittel benötigt. Da der Träger, insbesondere das Salz, ebenfalls natürlichen Ursprungs ist, und die weiteren Inhaltsstoffe (Farbstoffe, Lipide, ggf. Zusatzstoffe) gleichfalls aus dieser Kategorie gewählt werden können (siehe nachfolgende Beschreibung), liegt insgesamt ein 2-Komponenten-Badezusatz auf natürlicher Basis vor, der aufgrund seiner Beschaffenheit und insbesondere der Abwesenheit eher schwer löslicher Retardierungsmittel (insbesondere in Form von Umhüllungsschichten) nahezu keine Rückstände in der Badewanne hinterlässt.

Die Zusatzwirkstoffe liegen bevorzugt in einer Gesamtmenge von 0,5 bis 5 Gew.%, ins-besondere 0,5 bis 2 Gew.% in der jeweiligen Komponente vor.

### 3.2. Galenische Zusatzstoffe (in Komponente 1, 2 insbesondere in 2)

Zu den galenischen Hilfsmitteln gehören beispielsweise Konditioniermittel wie Mono-und Dialkylphosphate oder Cetylalkohol o. ä. oder Mischungen hiervon, Dextrine, Kolloide, z.B. Polysaccharide (wie Xanthan Gum), Dextrine, Cyclodextrin oder ähnliche kolloide Saccharide, Lactose, Celluloseether, wie Methylcellulose, Hydroxyethylcellulose, Hydroxypropylcellulose, Eiweißhydrolysate ferner hochdisperse Kieselsäureverbindungen wie Siliciumdioxid (Silica), Aerosile, Zucker wie Lactose, Stärkeprodukte wie Reis-, Weizen-, Mais- und Kartoffelstärke (Tapioka), ferner auch hydrophobisch modifizierte Stärken, ferner Lösungsvermittler wie Ethanol, ggf. Wasser (dieses z. B. in geringen Mengen wie < 1 Gew.%, vor allem < 0,5 Gew.%, z. B. 0,1 Gew.%), Isopropanol, (Poly)-Ethylenglykol, (Poly)-Propylenglykol. Besonders bevorzugt sind hier natürliche Substanzen wie Zucker, z.B. Lactose, Stärke, Silica oder Mischungen hiervon.

Das oder die Konditioniermittel sind bevorzugt in Mengen von bis zu 3 Gew.% vorhanden. Das oder die Konditioniermittel können in beiden Komponenten des Badezusatzes, bevorzugt jedoch in der zweiten Komponente vorhanden sein, worin auch geringe Mengen Wasser vorhanden sein können wie z. B. kleiner 1 Gew.%, insbesondere kleiner 0,5 oder 0,1 Gew.%.

### 3.3. Reinigungstenside (Emulgatortenside) in Komponente 1

Emulgatortenside, die in geeigneten Mengen hinzugesetzt werden können, werden vor allem ausgewählt aus nicht ionischen Tensidemulgatoren, anionischen Tensidemulgatoren oder Mischungen hiervon. Insbesondere bevorzugt sind wasserlösliche anionische Tenside.

### 3.3.1. Aniontenside:

Hierzu gehören die bevorzugt gewählten anionischen, vor allem wasserlöslichen Fettalkoholtenside. Diese können auch mit nichtionischen Tensiden, wie nachfolgend beschriebenen nicht ionischen Fettsäure- bzw. Fettalkoholderivaten (ethoxylierte bzw. propoxylierte, vorzugsweise ethoxylierte Derivate) oder nichtionischen Fettsäureamiden kombiniert werden.

Zu den geeigneten Aniontensid- Fettalkoholtensiden gehören vor allem C₈ bis C₂₂-, vor allem C₁₀ bis C₁₈-Fettalkoholsulfate bzw. C₈₋₂₂-Alkylsulfate bzw. C₈₋₂₂ -Alkylethersulfate oder deren Sulfatsalze wie Aminsalze, insbesondere Monoisopropanolaminsulfatsalze (z. B. MIPA Laurethsulfat (Monoisopropanolamine-(EO1-4)- Laurylsulfat, ggf. ethoxylierte Derivate hiervon mit einem EO Grad von 0 bis 4, vor allem 1-4). Beispiele weiterer Salze sind Natriumlaurylsulfat, Natriumlaurylethersulfat.

Andere geeignete anionische wasserlösliche Tensid-Verbindungen sind (C₁-C₁₈- Alkyl)-C₁₂ -C₂₂-Fettalkohol-Aminosäureester-Verbindungen, insbesondere Salze hiervon, wobei die Aminosäuren ausgewählt sind aus Glycin, Taurin und anderen. Diese können als Salze eingesetzt werden wie z. B. Alkali-Erdalkalisalze (Na-/K-Ca/-, Mg-Chloride, -Sulfate,- Phosphate) wie vor allem wie Oleyl-, Lauryl- Taurate, z.B. Natriummethyloleyltaurat.

Ferner geeignet sind auch anionische C₁₂₋₂₂-Sulfo-Succinate wie z.B. Disodium Lauryl Sulfosuccinat (im Handel erhältlich z. B. als Plantaton® SUS).

### 3.3.2. Nicht ionische Tenside

Hierzu gehören wasserlösliche oder fettlösliche Tenside. Diese nichtionischen Tenside werden bevorzugt auf Fettsäure- oder Fettalkoholbasis gewählt wie nicht ionische ethoxylierte und/oder propoxylierte Fettalkohole (Fettalkoholether), nichtionische ethoxylierte und/ oder propoxylierte Fettsäuren (Ester) oder deren Amide wie z. B. Mono- oder Diethanolamide eingesetzt werden, wobei der Fettalkohol bzw. die Fettsäure eine Kettenlänge C₈ bis C₂₀, bzw. C₁₂ bis C_{22;} vor allem C₁₂ bis C₁₈ aufweist. Hierzu gehören vor allem C₁-C₈-Alkyl-C₁₀-C₁₈-Fettalkoholethoxylate mit einem Ethoxylierungsgrad (EO), einem Propoxylierungsgrad (PO) oder einem Gemischtoxylierungsgrad (EO/PO) zwischen 1 und 5 (und einem HLB- Wert von weniger als 10, vor allem weniger als 8) wie Polyoxyethylenlaurylether (1 bis 4 EO, Laureth 1-4), Polyoxyethylen (5)- Oleylether, Po-lyoxyethylen(7)palmitylether, Polyoxyethylen(15) stearylether. Bevorzugt sind ethoxylierte Produkte.

Dazu gehören weiterhin derartige nichtionische Tenside mit höherem Oxylierungsgrad und entsprechend höherem HLB- Wert von mehr als 10 wie von 11-40, vor allem 11-25, insbesondere 13 bis 20. Insbesondere zählen hierzu höher polyoxyethylierte/und/oder -propoxylierte C₈₋₂₂-Fettalkohole (vor allem Alkyl-C₁₀-C₁₈-Fettalkoholalkoxylate) oder derartige ethoxylierte und/ oder propoxylierte C₁₂₋₂₂-Fettsäuren mit Ethoxylierungs- und/ oder Propoxylierungsgraden von 8-25 oder Mischungen hiervon wie (HLB- Werte in Klammern): Polyoxypropylen-(15)-stearylether oder Polyoxyethylensorbitanmonooleat (15,0), Polyoxyethylensorbitanmonostearat - oder -palmitat (15,0), Polyoxyethylenstearylether (15,3), Polyoxyethylenoleyether (15,3), Polyoxyethylenoxypropylenmonostearat (15,7), Polyoxyethylenfettalkoholether (16,0), Polyoxyethylenmonostearat (HLB 15) u.ä.. Insbesondere bevorzugt sind hier ethoxylierte C₁₂₋₂₂-Fettsäuren oder ethoxylierte C₈₋₂₂-Fettalkohole mit Ethoxylierungsgraden von 10-20 (HLB- Wert z. B. von 13 bis 20, vor allem 13,5 bis 18). Hierzu gehören z.B. die Polyoxyethylenether des Laurylalkohols (Laureth 4, HLB 9,7; Laureth-23, HLB 16,9), welche unter dem Handelsnamen Brij® bekannt sind, oder auch die Polyoxyethylenether des Cetylalkohols wie Ceteth-2 (HLB 5), Ceteth 10 (HLB 12,9), Ceteth-20 (Cetomacrogol 1000, HLB 15,7), oder auch Cetyl-Stearylether wie Ceteareth-6,20,25 (Cremophor-Produkte, HLB 10-17) u.a..

Ferner geeignet sind hier auch Amide der Fettsäuren oder der ethoxylierten Fettsäuren einer Kettenlänge C₈ bis C₂₀, vor allem C₁₂ bis C₁₈, wie Ethanolamide oder Diethanolamide wie z. B. Kokosfettsäurediethanolamid.

Ebenso geeignet sind nichtionische C₁₂ -C₂₂-Fettsäureether und C₁₂ -C₂₂-Fettsäureester von mehrwertigen Alkoholen wie Glycerol, Sorbitan, wie Mono-, Di-und Triglyceride von C₁₂ -C₂₂-Fettsäuren (Glyceridester) oder C₁₂ -C₂₂-Fettalkoholen (Glyceridether), Mono-, Di- und Tri-/tetra- usw. Sorbitan-C₁₂ -C₂₂-Fettsäureester oder Sorbitan-C₁₂-C₂₂-Fettalkoholether, welche ggf. auch oxyliert (ethoxyliert/propoxyliert, gemischtoxyliert) sein können. Beispiele hierzu sind Glyceryllaureate, Polysorbat (z. B. Tween 20, Tween 60, Tween 21).

Eine weitere Gruppe geeigneter nichtionischer Tenside stellen Zuckerester oder Zuckerether von Glucose, Saccharose, Methylglukose von C₁₂-C₂₂ -gesättigten, -ungesättigten, -partial gesättigten Fettsäuren; oder von polyoxyethylierten und/ oder polyoxypropylierten mittelkettigen und höherkettigen C₁₂ -C₂₂ -Fettsäuren (Ester) dar; bzw. von C₈-C₂₀- gesättigten, -ungesättigten, -partial gesättigten oder derartigen polyoxyethylierten und/oder polyoxypropylierten Fettalkoholen (Ether). Bei polyoxyethylierten oder polyoxypropylierten Produkten kann der EO (PEG- Produkte) bzw. PO (PPG-Produkte)- Grad zwischen 8 und 40, vorzugsweise 8-25 liegen. Es können auch gemischt polyoxylierte Produkte eingesetzt werden.

Als Zucker eignen sich insbesondere Glukose, Saccharose, Methylglukose. Als Fettsäuren bzw. Fettalkohole sind vor allem organische aliphatische C₁₂-C₂₂-Carbonsäuren bzw. derartige Alkohole geeignet. Hierzu gehören insbesondere Caprylsäure, Caprinsäure, Laurinsäure, Myristinsäure, Palmitinsäure, Stearinsäure, Ölsäure bzw. derartige Alkohole wie Laurylalkohol, Stearylalkohol und auch Mischungen dieser.

Bekannte oxylierte (ethoxylierte, propoxylierte, gemischtoxylierte) Zuckertenside sind z. B. PEG-20 Methylglucose-Sesquistearat, PEG-120-Methlyglucosedioleat bzw. Methyl-Gluceth-10, Methyl-Gluceth-20, PPG-10-Methylglucoseether, PPG-20-Methyl-glucoseether.

Der oder die Emulgatortenside, welche in der ersten Komponente des Kombinations-Badezusatzes vorhanden sein können, werden bevorzugt in geringen Mengen eingesetzt, wie 0,01 bis 1 Gew.% , vorzugsweise 0,01 bis 0,5 Gew.%. Als wasserlösliche Tenside werden vorzugsweise anionische Substanzen ausgewählt, wie (C₁-C₁₈- Alkyl)-C₁₂ -C₂₂-Fettalkohol-Aminosäureester-Verbindungen (Taurate), Alkylsulfate, Alkylethersulfate wie beschrieben oder auch nicht ionische Tenside wie C₁₂ -C₂₂-Fettsäureether und C₁₂ -C₂₂-Fettsäureester von mehrwertigen Alkoholen wie -Laurylalkoholen-, und insbesondere Laureth 4 (Brij- Produkte).

### 4. Trägersubstanzen

Als Trägersubstanzen kommen vor allem Salze infrage, insbesondere für beide Komponenten, wobei in beiden Komponenten jeweils die gleichen Salze oder verschiedene Salze oder Salzgemische vorliegen können. Ganz besonders bevorzugt ist Meersalz, auch für beide Komponenten.

Als Salze können alle für diesen Zweck bekannten Salze verwendet werden wie Mineralsalze, insbesondere Salze aus den Kationen Natrium, Kalium, Magnesium, Calcium, Eisen, Ammonium oder Mangan und den Anionen Chlorid, Fluorid, Sulfat oder Nitrat als solche oder als Mischungen, wobei vor allem Natriumchlorid als Hauptanteil eingesetzt wird wie z. B. Solesalz, Meersalz. Möglich ist auch Totes Meer-Salz. Gegebenenfalls können auch andere aus dem Thermalbadmilieu bekannte Salze verwendet werden, welche auch die Kationen Lithium, Aluminium, Strontium, und die Anionen Iodid, Bromid, Thiosulfat, aufweisen. Hierdurch können thermalbadanaloge Bedingungen erzielt werden.

Es ist auch möglich, einen Teil des Mineralsalzes oder das Mineralsalz (nahezu) vollständig zu ersetzen durch Carbonatsalze (Brausesatzsalz), mit welchen bei der Anwendung durch die CO₂-Entwicklung noch zusätzlich ein Sprudeleffekt erzielt werden.

Bei Wahl eines Brausesatzsalzes umfasst dieser üblicherweise Säuren/Säureregulatoren wie beispielsweise Zitronensäure, Weinsäure, Fumarsäure, Adipinsäure; Säureanhydride, ausgewählt aus Zitronensäure-, Bernsteinsäureanhydrid oder deren sauren Salzen, ausgewählt aus Natriumdihydrogenphosphat, Natriumfumarat oder deren Mischungen. Diese können gegebenenfalls auch als galenische Hilfsmittel gelten bzw. eingesetzt werden.

Vorzugsweise besteht der Brausesatz hierbei aus Natrium(bi)-, Calcium-, MagnesiumAmmonium- oder Kalium(bi)carbonat oder deren Mischungen und einer oder mehrerer Säuren, die ausgewählt sind aus Zitronensäure, Weinsäure, Fumarsäure, Adipinsäure; Säureanhydride, ausgewählt aus Zitronensäure-, Bernsteinsäureanhydrid oder deren sauren Salzen, ausgewählt aus Natriumdihydrogenphosphat, Natriumfumarat oder deren Mischungen.

Dem Fachmann sind derartige Brausesätze bekannt (siehe EP 0930064).

Ganz besonders bevorzugt, vor allem auch als Träger für beide Komponenten, sind Meersalz, Totes Meersalz, Brausesatzsalz, Solesalz oder geeignete Mischungen hiervon. Insbesondere bevorzugt ist Meersalz, vor allem natürliches, oder auch raffiniertes oder teilraffiniertes Meersalz mit einem Hauptanteil an Natriumchlorid wie 85 bis 99%. Weiterhin bevorzugt ist Meersalz im Gemisch mit 10 bis 80% (bezogen auf den Anteil an Meersalz) an Brausesatzsalz.

Die Träger, insbesondere Salze, werden als Pulver oder Granulat für die Komponente 1 direkt eingesetzt und für die Komponente 2 in dieser Form verwendet zur Herstellung der festen, insbesondere gepressten Matrix.

Alternativ können auch wasserlösliche Träger aus der Gruppe, umfassend wasserlösliche Stoffe, ausgewählt aus Kollagenhydrolysat (z. B. mit einer mittleren molaren Masse von maximal 50 000 D, erhältlich aus pflanzlichen, tierischen oder marinen Quellen); Cellulose mit einer mittleren Molekülmasse von 50 000 bis 500 000 D, vorzugsweise 50 000 bis 250 000 D; Kohlenhydrate wie vor allem Saccharose, Fructose, Dextrose, Lactose, Sucrose, Glucose; Stärke (wie z. B. Reis-, Weizen-, Mais- und Kartoffelstärke), oder Harnstoff für die Trägerkomponente 1 jeweils mit einem Salz für die Trägerkomponente 2 kombiniert werden.

Darüberhinaus können die Trägerpartikel der ersten Komponente ausgewählt sein aus einem oder mehreren Salzen und die Träger der zweiten Komponente aus einem oder mehreren anderen Salzen.

Die Menge an Träger, vor allem Salzträger, in den erfindungsgemäßen Badezusätzen beträgt insgesamt im allgemeinen 96 bis 99 Gew.% (Komponente 1), insbesondere 90 bis 97 Gew.%, vor allem 94 bis 96,8 Gew.% (Komponente 2), bezogen auf die Gesamtzubereitung einer Komponente 1 bzw. 2.

In erfindungsgemäßen 2-Komponenten- Badezusätzen werden vor allem folgende Träger verwendet:

| | | | | |
|---|---|---|---|---|
| i) | Komp. 1: | Meersalz; | Komp. 2: | Meersalz; (jeweils bevorzugt natür-lich); |
| ii) | Komp. 1: | Meersalz; | Komp. 2: | Solesalz; |
| iii) | Komp. 1: | Dextrose, Sacharose | Komp. 2: | Meersalz, Solesalz oder Gemische hiervon; |
| v) | Komp. 1: | Totes Meersalz; | Komp. 2: | Meersalz; |
| v) | Komp. 1: | Kartoffel-Weizenstärke | Komp. 2: | Meersalz; |
| vi) | Komp. 1: | Solesalz | Komp. 2: | Meersalz |
| vii) | Komp. 1: | Harnstoff | Komp. 2: | Totes Meersalz |
| viii) | Komp. 1: | Zucker (z.B. Glucose, Sucrose, oder Gemische hiervon); | Komp: 2: | Meersalz, Solesalz oder Gemische hiervon |
| ix) | Komp. 1: | Meersalz | Komp. 2: | Totes Meersalz. |

Besonders bevorzugte Kombinationen sind die unter i), ii), v), vii),viii) oder auch ix) genannten.

### Herstellung

Die erfindungsgemäßen Zubereitungen werden vorzugsweise wie folgt hergestellt:
1) Zunächst wird in einem geeigneten Mischer wie Pflugscharmischer die Komponente 1 hergestellt, indem auf die vorgelegten pulver-oder granulatförmigen Trägerpartikel 1, insbesondere Salze, die Wirkstoffgruppe 1, insbesondere der/die Farbstoffe, ggf. einschl. Zusatzstoffe wie vor allem Emulgatortensid, Zusatzwirkstoffe wie Aromen, Vitamin/e, Lipide aufgetragen bzw. gemischt wird. Dabei wird ein körniges loses Pulver oder Granulat je nach Korngröße des Trägers erhalten. Dem Fachmann sind die dazu erforderlichen Mittel/Vorrichtungen/Bedingungen bekannt.
2) Anschließend werden die pulver-oder granulatförmigen Trägerpartikel 2, insbesondere das Trägersalz der Komponente 2 zusammen mit der Wirkstoffgruppe 2, nämlich dem/den (anderen) Farbstoff/en, dem flüssigen Öl und ggf. Zusatzstoffen, insbesondere Konditioniermitteln, in einem geeigneten Mischer gemischt bzw. aufgetragen und sodann vorzugsweise zu einer Tablette verpresst. Dem Fachmann sind die dazu erforderlichen Mittel/Vorrichtungen/Bedingungen bekannt.
3) Die gemäß Schritt 1) bzw. Schritt 2) erhaltenen Formkörperkomponenten 1 bzw. 2 werden zusammen als Mischung im Verhältnis 5:1 bis 1:1 in geeignete Behälter wie z. B. Einmal-Sachets oder größere Gebinde abgefüllt.

Die Herstellung des Produktes erfolgt im allgemeinen bei Raumtemperatur.

### Anwendung und Anwendungsformen

Die erfindungsgemäßen Zubereitungen werden als trockene Feststoffmischung aus losem Formkörper 1 in Form von Pulver, bevorzugt als Granulat, zusammen mit den gepressten festen Formkörpern 2, insbesondere Tabletten aus Salz, in geeigneten Kombinationsmengen von insgesamt 50 bis 100 g wie 60, 65, 80, 85, 100 g Gesamtmenge bereitgestellt. Bevorzugt sind Einzelgebinde, z. B. enthaltend 60 bis 80 g Mischung 3:1 oder 4:1 lose Formkörper 1, insbesondere Salzpulver/Salzgranulat zu gepresstem Formkörper 2 wie Tablette). Geeignete Behältnisse für den erfindungsgemäßen Kombinations-Badezusatz sind z. B. Sachets verschiedener Formen wie länglich, rechteckig, oval, aus flexiblen Materialien wie insbesondere undurchsichtiger und transparenter Folie, geschlossen oder auch perforiert, z. B. in Form von flexiblen Kissen, Schlauchbehältnissen u.ä., die aus geeigneten Materialien wie Baumwolle, Gaze, oder aus anderem, für derartige Zwecke geeignetem und bekanntem textilem Kunststoffgewebe bestehen. Die Anwendung von Sachets aus transparenter oder teilweise transparenter Folie führt sogleich zur optischen Wahrnehmung der unterschiedlich gefärbten einzelnen Trägerpartikel wie z.B. gelbe Salzkristalle (Komponente 1) + rote Tabletten (Komponente 2). Die Partikel der Komponente 2, insbesondere Tabletten, können den ca. 1 bis 10 fachen Durchmesser, vorzugsweise den 1,5 bis 7-fachen Durchmesser, des Durchmessers der Partikel der Komponente 1 (Salzkristalle) aufweisen. Denkbar sind auch Packungen, z. B. aus Karton, welche mehrere Einzelsachets enthalten.

Aus dem jeweiligen Gebinde wird die geeignete Menge, welche in Einzelgebinden vorgegeben ist (z. B. 50 bis 100 g), in die Badeflotte auf einmal eingetragen. Es entfaltet sich zunächst die Farbe der Formkörperkomponente 1, ggf. zusammen mit dem Tensid, und sodann nach einigen Minuten (z.B. 3-5 Minuten) die vorzugsweise darauf abgestimmte Färbung der Komponente 2. Optional können dabei auch Aromen oder ggf. unterschiedliche Aromen in der ersten und/oder der 2. Farbphase mit freigesetzt werden. Durch eine gezielte Farbkombination und ggf. auch Aromakombination kann somit eine visuelle Wirkung erzielt werden, welche die durch die Trägerstoffe, insbesondere Salze hervorgerufene Wirkung unterstützt. Diese kann weiterhin verstärkt werden durch Zusatz dafür bekannter Wirkstoffe wie beschrieben.

Der Badezusatz ist leicht löslich. Aufgrund des Fehlens von auf den Partikeln aufgetragenen umhüllenden Retardierungsmitteln können die durch nicht gelöste Stoffe bedingten Anhaftungen in der Wanne vermieden werden.

Besonders bevorzugte Anwendungsformen sind undurchsichtige längliche Sachets mit Badezusätzen zur Herbeiführung eines entspannenden Sonnenaufgang Gelb-Orange-Rot-Farbeffekts (Sonnenaufgang), oder mit beruhigendem Blau-Grüneffekt (Wald-Lagune) oder mit durchwärmenden Rot-Violett-Effekt. Dabei können optional geeignete Aromen hinzugesetzt werden wie Orangenöl, Minzöl, Melissenöl, Lavendelöl. Mittels geeigneter Farb- und Zusatzwirkstoffe können entsprechende Indikationen wie Entspannung, Belebung, Muskel-und Gelenkwirkungen, Durchwärmung, Atmung (Erkältung) u.a. bekannte Wirkungen erzielt werden. Der Badezusatz kann als Vollbad in der Wanne, oder als Teilbad (Sitzbad, Fußbad) eingesetzt und verwendet werden, wobei oben beschriebene Wirkungen erzielt werden können.

Andere Darreichungs- bzw. Anwendungsformen sind ebenfalls möglich und erfindungsgemäß umfasst.

### Beispiele

Die Erfindung wird anhand der nachfolgenden Beispiele näher erläutert.

### Beispiel 1

### Beispiel 1a) Komponente 1: Loses Badesalz

| Inhaltsstoff | Gew.% |
|---|---|
| Meersalz | 98,88% |
| Parfümöl | 0,50% |
| Chinolingelb | 0,30% |
| Disodium Lauryl Sulfosuccinate | 0,20% |
| Tocopherol | 0,02% |
| Macadamianussöl | 0,10% |
| | 100,00% |

### Beispiel 1b) Komponente 2: gepresste Tablette

| **Inhaltsstoff** | **Gew.%** |
|---|---|
| Meersalz | 95,80% |
| Lactose | 2,50% |
| Distelöl | 1,00% |
| Cochenillerot | 0,50% |
| Silica | 0,10% |
| Wasser | 0,10% |
| | 100,00% |

### Beispiel 2

### Beispiel 2a) Komponente 1: Loses Badesalz

| **Inhaltsstoff** | **Gew.%** |
|---|---|
| Meersalz | 99,365% |
| Parfümöl | 0,100% |
| Chinolingelb | 0,165% |
| Disodium Lauryl Sulfosuccinate | 0,150% |
| Kakaobutter | 0,020% |
| Macadamianussöl | 0,200% |
| | 100,000% |

### Beispiel 2b) Komponente 2: gepresste Tablette

| **Inhaltsstoff** | **Gew.%** |
|---|---|
| Meersalz | 95,950% |
| Lactose | 2,100% |
| Reiskeimöl | 1,250% |
| Cochenillerot | 0,500% |
| Silica | 0,100% |
| Wasser | 0,100% |
| | 100,000% |

### Beispiel 3

### Beispiel 3a) Komponente 1: Loses Badesalz

| **Inhaltsstoff** | **Gew.%** |
|---|---|
| Harnstoff | 99,43% |
| Parfümöl | 0,150% |
| Cochenillerot | 0,050% |
| Sodium Methyl Oleoyl Taurate | 0,100% |
| Tocopherol | 0,050% |
| Sheabutter | 0,020% |
| Calendulaextrakt | 0,100% |
| Macadamianussöl | 0,100% |
| | 100,00% |

### Beispiel 3b) Komponente 2: gepresste Tablette

| **Inhaltsstoff** | **Gew.%** |
|---|---|
| Meersalz | 96,79% |
| Maisstärke | 2,00g% |
| Mandelöl | 1,00% |
| Patentblau | 0,01% |
| Silica | 0,10% |
| Wasser | 0,10% |
| | 100,00% |

### Beispiel 4

### Beispiel 4a) Komponente 1: Loses Badesalz

| **Inhaltsstoff** | **Gew.%** |
|---|---|
| Totes Meersalz | 99,50% |
| Parfümöl | 0,20% |
| Chinolingelb | 0,10% |
| Laureth-4 | 0,15% |
| Coenzym Q10 | 0,05% |
| | 100,00% |

### Beispiel 4b) Komponente 2: gepresste Tablette

| Inhaltsstoff | **Gew.%** |
|---|---|
| Meersalz | 96,79% |
| Xanthan Gum | 1,50% |
| Avocadoöl | 1,50% |
| Patentblau | 0,01% |
| Silica | 0,10% |
| Wasser | 0,10% |
| | 100,00% |

### Beispiel 5

### Beispiel 5a) Komponente 1: Loses Badesalz mit Brausesatz

| Inhaltsstoff | **Gew.%** |
|---|---|
| Meersalz | 69,60% |
| Natriumbicarbonat | 15,00% |
| Zitronensäure | 15,00% |
| Rosmarinöl | 0,15% |
| Pfefferminzöl | 0,05% |
| Chinolingelb | 0,10% |
| Sodium Methyl Oleoyl Taurate | 0,10% |
| | 100,00% |

### Beispiel 5b) Komponente 2: gepresste Tablette

| Inhaltsstoff | Gew.% |
|---|---|
| Meersalz | 97,29% |
| Maisstärke | 1,75% |
| Nachtkerzenöl | 0,75% |
| Patentblau | 0,01% |
| Silica | 0,10% |
| Wasser | 0,10% |
| | 100,00% |

### Beispiel 6

### Beispiel 6a) Komponente 1: Loses Badesalz

| Inhaltsstoff | Gew.% |
|---|---|
| Steinsalz | 99,50% |
| Baldrianöl | 0,05% |
| Citronellöl | 0,10% |
| Cochenillerot | 0,15% |
| Disodium Lauryl Sulfosuccinate | 0,20% |
| | 100,00% |

### Beispiel 6b) Komponente 2: gepresste Tablette

| Inhaltsstoff | Gew.% |
|---|---|
| Meersalz | 97,04% |
| Lactose | 2,00% |
| Hagebuttenkernöl | 0,75% |
| Patentblau | 0,01% |
| Silica | 0,10% |
| Wasser | 0,10% |
| | 100,00% |

## Patentansprüche

1. Fester, 2- Komponenten-Kombinations-Badezusatz auf Basis eines wasserlöslichen Trägers, **dadurch gekennzeichnet, dass** er besteht aus:
- einer Formkörperkomponente 1 mit loser schüttbarer Matrix, umfassend mehr als 70 Gew.% eines wasserlöslichen Trägers 1 und als Rest eine Wirkstoffgruppe 1, umfassend einen oder mehrere Farbstoffe, sowie ggf. Zusatzstoffe;
- einer Formkörperkomponente 2 mit fester gepresster Matrix, umfassend mehr als 70 Gew.% eines wasserlöslichen Trägers 2 und als Rest eine Wirkstoffgruppe 2, umfassend ein oder mehrere Lipide, einen oder mehrere, vom in der Formkörperkomponente 1 vorhandenen Farbstoff/en verschiedene(n) Farbstoff(e) und ggf. Zusatzstoffe, wobei der Mengenanteil an Formkörperkomponente 1 zu Anteil Formkörperkomponente 2 im Kombinations-Badezusatz 5:1 bis 1:1 beträgt mit der Maßgabe, dass mindestens ein Lipid in der Komponente 2 ein flüssiges Öl ist.

2. Badezusatz gemäß Anspruch 1, **dadurch gekennzeichnet, dass** die Formkörperkomponente 1 mit loser Matrix 90 bis 99,5 Gew.% des wasserlöslichen Trägers 1 und 0,5 bis 10 Gew.% der Wirkstoffgruppe 1 umfasst und die Formkörperkomponente 2 mit gepresster Matrix 90 bis 98 Gew.% des wasserlöslichen Trägers 2 und 10 bis 2 Gew.% der Wirkstoffgruppe 2 umfasst.

3. Badezusatz nach einem der Ansprüche 1 oder 2, **dadurch gekennzeichnet, dass** in der Formkörperkomponente 1 ein oder mehrere Zusatzstoffe, ausgewählt aus Emulgatortensiden, Konditioniermitteln, Zusatzwirkstoffen aus der Gruppe aus ätherischen Ölen/Extrakten, Vitaminen, Lipiden oder Mischungen hiervon, vorhanden sind.

4. Badezusatz nach einem der Ansprüche 1 bis 3, **dadurch gekennzeichnet, dass** in der Formkörperkomponente 2 ein oder mehrere Zusatzstoffe, ausgewählt aus Pflegewirkstoffen oder Vitaminen, Konditioniermitteln vorhanden sind.

5. Badezusatz nach einem der Ansprüche 1 bis 4, **dadurch gekennzeichnet, dass** der wasserlösliche Träger 1 der Formkörperkomponente 1 ausgewählt ist aus Salzen, Harnstoff, Kollagenhydrolysat, Cellulose, Kohlenhydraten wie Saccharose, Fructose, Dextrose, Lactose, und der wasserlösliche Träger 2 ausgewählt ist aus Salzen.

6. Badezusatz nach einem der Ansprüche 1 bis 5, **dadurch gekennzeichnet, dass** die Träger 1, 2 der Formkörperkomponente 1, 2 ausgewählt sind aus Meersalz, Totes Meersalz, Solesalz, Mineralsalz, Siedesalz oder Mischungen hiervon.

7. Badezusatz nach einem der Ansprüche 1 bis 6, **dadurch gekennzeichnet, dass** das flüssige Öl in der Formkörperkomponente 2 ausgewählt ist aus Distelöl, Reiskeimöl, Mandelöl, Sojaöl oder Mischungen hiervon.

8. Badezusatz nach einem der Ansprüche 1 bis 7, **dadurch gekennzeichnet, dass** die Formkörperkomponente 1 ein oder mehrere Vitamine, und ein oder mehrere feste Lipide, ein oder mehrere flüssige Lipide oder Mischungen hiervon aufweist.

9. Badezusatz nach einem der Ansprüche 1 bis 8, **dadurch gekennzeichnet, dass** in der Formkörperkomponente 1 ein oder mehrere anionische Emulgatortenside, ausgewählt aus C₁₀- bis C₁₈-Fettalkoholsulfaten, C₈₋₂₂-Alkylsulfaten, C₈₋₂₂-Alkyl-ethersulfaten oder deren Salzen, (C₁-C₁₈- Alkyl)-C₁₂-C₂₂-Fettalkohol-Aminosäureester-Verbindungen, insbesondere Salze hiervon, wobei die Aminosäuren ausgewählt sind aus Glycin, Taurin, ferner C₁₂₋₂₂-Sulfo-Succinate, oder Mischungen hiervon, vorhanden sind.

10. Badezusatz nach einem der Ansprüche 1 bis 9, **dadurch gekennzeichnet, dass** in der Formkörperkomponente 2 ein oder mehrere Konditioniermittel, ausgewählt aus Lactose, Silica und Stärke, vorhanden sind.

11. Badezusatz nach einem der Ansprüche 1 bis10, **dadurch gekennzeichnet, dass** die Wirkstoffkomponente 1 auf den Träger 1 der Formkörperkomponente 1 oberflächlich aufgetragen und die Wirkstoffkomponente 2 in den Träger 2 der Formkörperkomponente 2 eingepresst sind.

12. Badezusatz nach einem der Ansprüche 1 bis 11, **dadurch gekennzeichnet, dass** die Formkörperkomponente 1 als Pulver oder Granulat und die Formkörperkomponente 2 als Tablette vorliegt.

13. Badezusatz nach einem der Ansprüche 1 bis 12, **dadurch gekennzeichnet, dass** der Durchmesser der Partikel der Komponente 2, insbesondere der Tabletten, um das ca. 1 bis 10- Fache größer ist als der Durchmesser der Partikel der Komponente 1 (Salzkristalle).

14. Verfahren zur Herstellung eines Badezusatzes gemäß einem der Ansprüche 1 bis 13, **dadurch gekennzeichnet, dass** man
1) in einem geeigneten Mischer wie Pflugscharmischer die Komponente 1 herstellt, indem man auf die vorgelegten losen pulver- oder granulatförmigen Trägerpartikel 1, insbesondere Salze, die Wirkstoffgruppe 1, mit dem/den Farbstoff(en), sowie Zusatzstoffen sofern vorhanden, wie vor allem Emulgatortensid, Vitamin/en, Lipid/en, oberflächlich aufträgt; und das erhaltene Pulver/Granulat ggf. zu der gewünschten Korngröße vermahlt;
2) die pulvrigen oder granulatförmigen Trägerpartikel 2, insbesondere Trägersalz 2, der Komponente 2 zusammen mit der Wirkstoffgruppe 2, nämlich dem/den (anderen) Farbstoff/en, dem oder den Lipiden, insbesondere dem/den flüssigen Öl(en) und ggf. Zusatzstoffen, insbesondere Konditioniermitteln, in einem Pflugscharmischer oberflächlich aufträgt und die erhaltene Mischung sodann, vorzugsweise zu einer Tablette, verpresst;
3) die gemäß Schritt 1) bzw. Schritt 2) erhaltenen Formkörperkomponenten 1 bzw. 2 zusammen als Mischung im Verhältnis 5:1 bis 1:1 in geeignete Behälter wie z. B. Einmal-Sachets oder größere Gebinde abfüllt.

15. Verwendung einer Formkörperkomponente 1 mit loser schüttbarer Matrix, umfassend mehr als 70 Gew.% eines wasserlöslichen Trägers 1 und als Rest eine Wirkstoffgruppe 1, umfassend einen oder mehrere Farbstoffe, ggf. ein oder mehrere Zusatzstoffe; sowie einer Formkörperkomponente 2 mit fester gepresster Matrix, umfassend mehr als 70 Gew.% eines wasserlöslichen Trägers 2 und als Rest eine Wirkstoffgruppe 2, umfassend ein oder mehrere Lipide, einen oder mehrere, vom in der Formkörperkomponente 1 vorhandenen Farbstoff/en verschiedene(n) Farbstoff(e) und ggf. Zusatzstoffe, zur Herstellung eines Kombinations-Badezusatzes mit definiertem Farbwechsel bei der Anwendung, wobei der Mengenanteil an Formkörperkomponente 1 zu Formkörperkomponente 2 im Kombinations-Badezusatz 5:1 bis 1:1 beträgt und mindestens ein flüssiges Lipid, insbesondere ein flüssiges Öl, vorhanden ist.

16. Verwendung gemäß Anspruch 15, **dadurch gekennzeichnet, dass** die Formkörperkomponente 1 ein schüttbares Pulver oder Granulat aus Meer-, Mineral-, Siede-Solesalz oder Mischungen hiervon und die Formkörperkomponente 2 eine gepresste Tablette aus Meer-, Mineral-, Siede- Solesalz oder Mischungen hiervon ist.
